# EUROPEAN PATENT SPECIFICATION

(11) **EP 2 468 256 B1**
(45) Date of publication and mention of the grant of the patent: **21.08.2013**
(21) Application number: 11194783.4
(22) Date of filing: 21.12.2011
(51) Int. Cl.: A61K 9/00, A61K 9/20, A61K 31/33, A61P 3/10

(54) **Combinations of vildagliptin and glimepiride**
Kombinationen aus Vildagliptin und Glimepirid
Combinaisons de vildagliptine et de glimépiride

(30) Priority: 21.12.2010 TR 201010683; 24.02.2011 TR 201101809
(43) Date of publication of application: 27.06.2012
(73) Proprietor: Sanovel Ilaç Sanayi Ve Ticaret Anonim Sirketi, 34460 Sariyer/Istanbul (TR)
(72) Inventor: Cifter, Ümit, 34398 Istanbul (TR); Türkyilmaz, Ali, 34398 Istanbul (TR); Karaköy, Basak Acar, 34398 Istanbul (TR); Saydam, Mehtap, 34398 Istanbul (TR); Ergenekon, Ercan, 34398 Istanbul (TR)
(74) Representative: Sevinç, Erkan

(56) References cited:
- WO-A1-2006/094942
- WO-A1-2008/028914
- WO-A1-2010/107610
- WO-A1-2010/119990
- WO-A2-2010/092163
- GARBER A J ET AL: "Effects of vildagliptin on glucose control in patients with type 2 diabetes inadequately controlled with a sulphonylurea", DIABETES, OBESITY AND METABOLISM, BLACKWELL SCIENCE, OXFORD, GB, vol. 10, no. 11, 1 November 2008 (2008-11-01), pages 1047-1056, XP009149673, ISSN: 1462-8902, DOI: DOI:10.1111/J.1463-1326.2008.00859.X

## Description

### Field of Invention

The present invention relates to combinations comprising vildagliptin or a pharmaceutically acceptable salt of vildagliptin and glimepiride or a pharmaceutically acceptable salt of glimepiride.

### Background of Invention

Vildagliptin is a dipeptidyl dipeptidase-IV (DPP-IV) inhibitor developed for use in the treatment of type 2 diabetes (non-insulin dependent diabetes). Vildagliptin inhibits the degradation of the dipeptidyl dipeptidase-IV enzyme, thereby inhibiting the effects of incretin hormones, glucagon-like peptide-1 (GLP-1), and of glucose-dependent insulinotropic peptide (GIP). The chemical designation of vildagliptin is (S)-{[(3-hydroxyadamantan-1-yl)amino]acetyllpyrrolidine-2-carbonitril, with the chemical structure illustrated below in Formula 1.

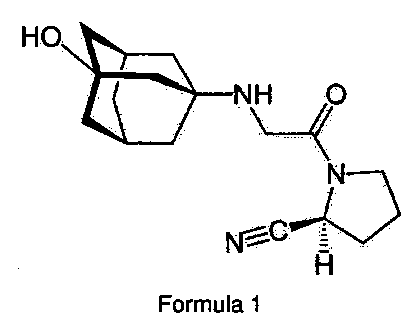

Vildagliptin is soluble in water and in organic polar solvents.

Vildagliptin is marketed under the trademark Galvus^{®} in 50 mg dosage forms. It is used against diabetes mellitus, but particularly in treating type 2 diabetes.

There are various patents available in the patent literature in relation to vildagliptin.

The patent application W00034241 discloses vildagliptin or an acid addition salt thereof, as well as their use in diabetes mellitus and obesity.

The patent application W02006078593 claims a direct-compression formulation of a DPP-IV inhibitor compound, and preferably of vildagliptin or an acid addition salt thereof.

The patent application W02006135723 discloses a formulation, comprising vildagliptin as an active agent, as well as hydroxypropyl methyl cellulose, microcrystalline cellulose, and magnesium stearate.

Glimepiride is an oral anti-diabetic drug with sulfonylurea structure. Glimepiride is used as an adjuvant to reduce blood sugar level in "diabetes mellitus type II" patients. As do all other sulfonylureas, it enhances the secretion of insulin from pancreatic beta cells and provides an insulin-like effect. The chemical designation of glimepiride is 1-({p-(2-(3-ethyl-4-methyl-2-oxo-3-pyrroline-1-carboxamide)ethyl)phenyl}sulfonyl)-3-(trans-4-methylcyclohexyl)urea, with the chemical structure illustrated below in Formula 2.

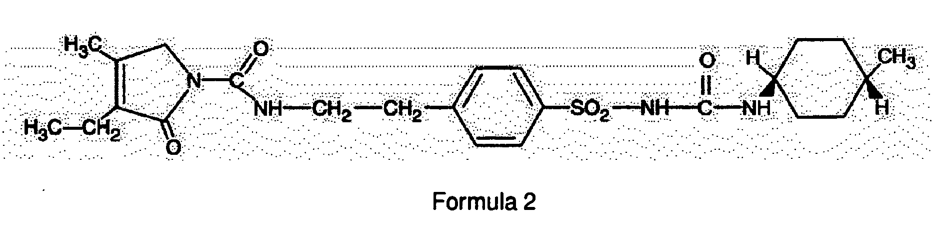

Glimepiride is marketed under the trademark AMARYL^{®} in 1, 2, and 4 mg dosage forms. It is used against diabetes mellitus, but particularly in treating type 2 diabetes.

The patent EP0031058 discloses the glimepiride molecule. The pharmaceutical composition thereof is indicated for diabetes at that document.

The patent EP0604853 discloses a formulation composed of glimepiride and a pharmaceutically acceptable excipient for use in treating arteriosclerosis.

The patent W02004082591 claims a formulation composed of milled glimepiride and excipients.

The patent W02007072218 discloses a formulation, composed of glimepiride with a particle size lower than 3 µm and of at least one excipient.

It is known that such drugs used in the same therapeutic field or even for the same symptoms are not brought always together with the expectation of additional therapeutic effects. There are many examples in the past, in which combinations of different classes used to treat the same indications have not yielded efficient or safe dosage forms resulting in different drug combinations. The cause of such unexpected incompatibility is diverse; the typically observed outcomes, however, include increase in side effects of different drug combinations, undesired drug interactions, and formation of new side effects.

There has been no pharmaceutical composition or dosage form produced until today, which contain the combination of vildagliptin and glimepiride.

Stability-related problems do occur in many active agents, including vildagliptin, under the influence of ambient and physical conditions. Vildagliptin, however, is an active agent that is highly-susceptible to air and humidity. When vildagliptin is exposed to air and humidity, it degrades structurally and develops chemical behavioral changes. The stability of vildagliptin products developed is not of a desired level and the shelf life thereof is shortened. Secondly, vildagliptin is reactive against the excipients employed in developing formulations containing vildagliptin. This fact causes impurities to occur in formulation and leads to incorporation of undesired components into the formulation.

In terms of vildagliptin and glimepiride, it is very important to prevent ambient influences and to block interaction with the exterior. Preventing ambient or environmental influences at a desired level, directly effects the stability and shelf life of a product. Its contact with water must not be avoided only efficiently during its production phase, but also when it is in a finished formulation form.

Whilst film coating is generally applied on finished formulations, this does not suffice in providing protection against humidity and therefore blisters, containing aluminum to avoid humidity, are used as well.

Carrying out the film coating process at a desired accuracy level is very crucial in terms of ensuring the stability of vildagliptin and glimepiride molecules. A preferred coating must both provide protection against environmental effects to ensure stability, and not lead to problems encountered in film coatings, such as wrinkling surfaces, blister and bubble formation, efflorescence, peeling, etc. The compatibility of a selected coating material with the active agent and excipients used in a formulation is also directly associated with the stability thereof.

Conventional diabetes therapies typically comprise administrating one or more different drug(s) as a pharmaceutical composition. Not all combinations of diabetes drugs, however, are as convenient as they are used individually, with respect to safety and efficiency. In result, a novelty is needed in the art of formulations, which can be used by diabetes patients.

### Object and Brief Description of Invention

The present invention provides a combination formulation of vildagliptin and glimepiride, eliminating all aforesaid problems and bringing additional advantages to the relevant prior art.

Accordingly, the main object of the present invention is to obtain a stable combination formulation for use in the diabetes disease.

Another object of the present invention is to obtain a combination of vildagliptin and glimepiride having desired levels of compatibility.

A further object of the present invention is to develop a coating, ensuring the stability of formulations comprising vildagliptin and glimepiride and not affecting the solubility and dissolution rates thereof.

Yet another object of the present invention is to obtain a combination of vildagliptin and glimepiride having desired levels of bioavailability.

Still another object of the present invention is to obtain a combination of vildagliptin and glimepiride having desired levels of dissolution rate and solubility.

Pharmaceutical combination formulations are realized to achieve all objects, referred to above and to emerge from the following detailed description.

In a preferred embodiment according to the present invention, said novelty is realized with vildagliptin or a pharmaceutically acceptable salt of vildagliptin, glimepiride or a pharmaceutically acceptable salt of glimepiride, and at least one coating layer.

In a preferred embodiment of the present invention, said coating layer is a sealed coating layer.

In a preferred embodiment according to the present invention, said sealed coating layer material comprises at least one or a properly-proportioned mixture of polyvinyl alcohol and hydroxypropyl methyl cellulose.

In a preferred embodiment according to the present invention, said coating further comprises at least one or a properly-proportioned mixture of xanthan gum and guar gum.

In a preferred embodiment according to the present invention, the weight of said coating layer with respect to the total weight of the formulation is 0.01 to 20% by weight, preferably 0.1 to 10% by weight, and more preferably 1 to 5% by weight.

In a preferred embodiment according to the present invention, said combination further comprises at least one or more than one excipient.

In another preferred embodiment according to the present invention, said excipients comprise at least one or a mixture of diluting agents, binders, disintegrants, surface-active agents, glidants, and lubricants.

In another preferred embodiment according to the present invention, said diluents include at least one or a mixture of lactose, microcrystalline cellulose, starch, mannitol, calcium phosphate anhydrate, calcium phosphate dihydrate, calcium phosphate trihydrate, dibasic calcium phosphate, calcium carbonate, calcium sulfate, carboxymethyl cellulose calcium, powdered cellulose, cellulose acetate, pregelatinized starch, lactose monohydrate, corn starch, with the preferred diluent being microcrystalline cellulose or lactose.

In another preferred embodiment according to the present invention, said binders include at least one or a mixture of polymethacrylate, glyceryl behenate, polyvinylpyrrolidone (povidone), hydroxypropyl methyl cellulose (HPMC), hydroxypropyl cellulose (HPC), carboxymethyl cellulose (CMC), methyl cellulose (MC), hydroxyethyl cellulose, sodium carboxymethyl cellulose (Na CMC), carboxymethyl cellulose calcium, ethyl cellulose and other cellulose derivatives, polyethylene oxide, gelatin, starch, xanthan gum, guar gum, alginate, carrageen, pectin, carbomer, cellulose acetate phthalate, hydroxypropyl starch, hydroxyethyl methyl cellulose, polaxomer, polyethylene glycol (PEG), with the preferred binder being polyvinylpyrrolidone.

In another preferred embodiment according to the present invention, said disintegrants include at least one or a mixture of croscarmellose sodium, sodium starch glycolate, cross-linked polyvinylpyrrolidone, starch, alginic acid, with the preferred disintegrator being croscarmellose sodium.

In another preferred embodiment according to the present invention, said surface-active agents include at least one or a mixture of sodium lauryl sulfate, polyoxyethylene glycol esters, sulfates comprising surface-active agents and poloxamer derivatives (sulfate and polaxomer derivatives comprising surface-active agents).

In another preferred embodiment according to the present invention, said glidants include at least one or a mixture of colloidal silicon dioxide, talk, aluminum silicate, powdered cellulose, calcium phosphate tribasic, hydrophobic colloidal silica, magnesium oxide, magnesium trisilicate, magnesium silicate, with the preferred glidant being colloidal silicone dioxide.

In another preferred embodiment according to the present invention, said lubricants include at least one or a mixture of calcium stearate, magnesium stearate, mineral oil, sodium stearyl fumarate, talk, polyethylene glycol, glyceryl monostearate, glyceryl palmitostearate, talk, zinc stearate, stearic acid, with the preferred lubricant being magnesium stearate.

In a further preferred embodiment according to the present invention, said formulation comprises cellulosic polymer in the core, and xanthan gum or guar gum in the coating layer. In a further preferred embodiment according to the present invention, said cellulosic polymer in said formulation is preferably hydroxypropyl methyl cellulose.

Another aspect of the present invention provides a method for preparing said pharmaceutical combination formulation, this method comprising the steps of
a. adding diluent into vildagliptin and glimepiride, stirring the resulting mixture,
b. adding polyvinylpyrrolidone and cross-linked polyvinylpyrrolidone to the resulting mixture and mixing the latter,
c. producing a wet granulation,
d. drying the granules,
e. sieving dried granules,
f. mixing the granules with magnesium stearate and silicium dioxide,
g. compressing the granules into tablets, and
h. film-coating the tablets with a water-soluble agent.

Still another aspect of the present invention provides another method for preparing said pharmaceutical combination formulation, this method comprising the steps of
a. adding lactose, polyvinylpyrrolidone, colloidal silicon dioxide, and magnesium stearate to vildagliptin and glimepiride, and stirring the resulting mixture,
b. compressing the granules into tablets, and
c. film-coating the tablets with a water-soluble agent.

### Detailed Description of Invention

### Example 1: Film-coated Tablet

### Wet Granulation

| **Ingredients** | **weight % (w/w)** |
|---|---|
| **core tablet** | |
| vildagliptin | 3.5 - 90 |
| glimepiride | 0.07 - 14.5 |
| polyvinylpyrrolidone | 0.25 - 10 |
| starch | 10 - 85 |
| cross-linked polyvinylpyrrolidone | 0.2 - 20 |
| colloidal silicon dioxide | 0.1 - 5 |
| magnesium stearate | 0.1 - 5 |

| **film tablet** | |
|---|---|
| hydroxypropyl methyl cellulose or polyvinyl alcohol, or a mixture thereof, or xanthan gum and guar gum | 0.1 - 5 |

In the tablet form according to the present invention, starch is added to vildagliptin and glimepiride, and the resulting mixture is stirred. Polyvinylpyrrolidone and cross-linked polyvinylpyrrolidone are added to and mixed together with the resulting mixture, thereby a wet granulation is formed. The formed granules are dried, sieved; thereafter colloidal silicon dioxide and magnesium stearate are added therein and mixed together. The formed granules are compressed into tablets. At the final step, tablets are subjected to film-coating.

### Example 2: Film-coated Tablet

| **Ingredients** | **weight % (w/w)** | **weight % (w/w)** |
|---|---|---|
| **core tablet** | | |
| vildagliptin | 29.4 | 3.5 - 90 |
| glimepiride | 4.7 | 0.07 - 14.5 |
| lactose monohydrate | 47.5 | 10 - 85 |
| microcrystalline cellulose | 12 | 2 - 90 |
| Na starch glycolate | 3 | 0.2 - 20 |
| croscarmellose sodium | 1 | 0.2 - 20 |
| Povidon K-25 | 1.4 | 0.25 - 10 |
| magnesium stearate | 1 | 0.1 - 5 |

| **film tablet** | | |
|---|---|---|
| hydroxypropyl methyl cellulose or polyvinyl alcohol, or a mixture thereof, or xanthan gum and guar gum | 0.1 - 5 | 0.1 - 5 |

In the tablet form made via wet granulation according to the present invention, vildagliptin, glimepiride, lactose monohydrate, sodium starch glycolate, microcrystalline cellulose are mixed together. Then, Povidon K-25 dissolved in water/alcohol is sprayed to this mixture in a fluidized bed granulator. Coated granules are dried and sieved, croscarmellose sodium is added, the resulting mixture is mixed, magnesium stearate is added and mixed. At the final step, the mixture is compressed into tablets and film-coated.

### Direct compression

| **Ingredients** | **weight % (w/w)** |
|---|---|
| **core tablet** | |
| vildagliptin | 3.5 - 90 |
| glimepiride | 0.07 - 14.5 |
| lactose (spray-dried) | 10 - 85 |
| cross-linked polyvinylpyrrolidone | 0.2 - 20 |
| colloidal silicon dioxide | 0.1 - 5 |
| magnesium stearate | 0.1 - 5 |

| **film tablet** | |
|---|---|
| hydroxypropyl methyl cellulose or polyvinyl alcohol, or a mixture thereof, or xanthan gum and guar gum | 0.1 - 5 |

In the direct-compression tablet form according to the present invention, lactose, polyvinylpyrrolidone, colloidal silicon dioxide, and magnesium stearate are added to vildagliptin and glimepiride, the resulting mixture is stirred and compressed into tablets. At the final step, tablets are subjected to film-coating.

With the present invention, combination formulations of glimepiride and vildagliptin are obtained, which have surprisingly good stability, solubility, and dissolution rate. The preferred coating layer materials are water soluble and quickly dissolves after swallowing. The seal coat provides a moisture barrier and hardness the surface of the tablet in order to minimize moisture and attritional effects. Glimepiride hydrochloride is preferably used in the formulation according to the present invention.

The treatment of diabetes disease is provided with the combination formulations developed. The protection scope of the present invention is set forth in the annexed claims and cannot be restricted to the illustrative disclosures given above, under the detailed description. Any alternative embodiments to be produced by those skilled in the art according to the basic principles, which are under the protection scope as set forth in the claims, shall be an infringement of the present invention.

## Claims

1. A pharmaceutical combination formulation, comprising vildagliptin or a pharmaceutically acceptable salt of vildagliptin, glimepiride or a pharmaceutically acceptable salt of glimepiride, and at least one coating layer.

2. The pharmaceutical combination formulation according to any of the preceding claims, wherein said coating layer is a sealed coating comprising at least one or a mixture of polyvinyl alcohol and hydroxypropyl methyl cellulose.

3. The pharmaceutical combination formulation according to any of the preceding claims, further comprising at least one or a mixture of xanthan gum and guar gum in the coating.

4. The pharmaceutical combination formulation according to any of the preceding claims, wherein the weight of said coating layer with respect to the total weight of the formulation is 0.01 to 20% by weight, preferably 0.1 to 10% by weight, and more preferably 1 to 5% by weight.

5. The pharmaceutical combination formulation according to any of the preceding claims, further comprising at least one or more than one excipient.

6. The pharmaceutical combination formulation according to any of the preceding claims, wherein said excipient is at least one or a mixture of diluting agents, binders, disintegrants, surface-active agents, glidants, and lubricants.

7. The pharmaceutical combination formulation according to any of the preceding claims, wherein said diluents include at least one or a mixture of lactose, microcrystalline cellulose, starch, mannitol, calcium phosphate anhydrate, calcium phosphate dihydrate, calcium phosphate trihydrate, dibasic calcium phosphate, calcium carbonate, calcium sulfate, carboxymethyl cellulose calcium, powdered cellulose, cellulose acetate, pregelatinized starch, lactose monohydrate, corn starch, with the preferred diluent being microcrystalline cellulose or lactose.

8. The pharmaceutical combination formulation according to any of the preceding claims, wherein said binders include at least one or a mixture of polymethacrylate, glyceryl behenate, polyvinylpyrrolidone (povidone), hydroxypropyl methyl cellulose (HPMC), hydroxypropyl cellulose (HPC), carboxymethyl cellulose (CMC), methyl cellulose (MC), hydroxyethyl cellulose, sodium carboxymethyl cellulose (Na CMC), carboxymethyl cellulose calcium, ethyl cellulose and other cellulose derivatives, polyethylene oxide, gelatin, starch, xanthan gum, guar gum, alginate, carrageen, pectin, carbomer, cellulose acetate phthalate, hydroxypropyl starch, hydroxyethyl methyl cellulose, polaxomer, polyethylene glycol (PEG), with the preferred binder being polyvinylpyrrolidone.

9. The pharmaceutical combination formulation according to any of the preceding claims, wherein said disintegrants include at least one or a mixture of croscarmellose sodium, sodium starch glycolate, cross-linked polyvinylpyrrolidone, starch, alginic acid, with the preferred disintegrant being croscarmellose sodium.

10. The pharmaceutical combination formulation according to any of the preceding claims, wherein said surface-active agents include at least one or a mixture of sodium lauryl sulfate, polyoxyethylene glycol esters, sulfates comprising surface-active agents and poloxamer derivatives (sulfate and polaxomer derivatives comprising surface-active agents).

11. The pharmaceutical combination formulation according to any of the preceding claims, wherein said glidants include at least one or a mixture of colloidal silicon dioxide, talk, aluminum silicate, powdered cellulose, calcium phosphate tribasic, hydrophobic colloidal silica, magnesium oxide, magnesium trisilicate, magnesium silicate, with the preferred glidant being colloidal silicone dioxide.

12. The pharmaceutical combination formulation according to any of the preceding claims, wherein said lubricants include at least one or a mixture of calcium stearate, magnesium stearate, mineral oil, sodium stearyl fumarate, talk, polyethylene glycol, glyceryl monostearate, glyceryl palmitostearate, talk, zinc stearate, stearic acid, with the preferred lubricant being magnesium stearate.

13. The pharmaceutical combination formulation according to any of the preceding claims, comprising further cellulosic polymer in the core and xanthan gum or guar gum in the coating layer.

14. The pharmaceutical combination formulation according to any of the preceding claims, wherein said cellulosic polymer is preferably hydroxypropyl methyl cellulose.

15. A method for preparing a pharmaceutical combination formulation according to any of the preceding claims, comprising the steps of
a. adding diluent into vildagliptin and glimepiride, mixing the resulting mixture,
b. adding polyvinylpyrrolidone and cross-linked polyvinylpyrrolidone to the resulting mixture and mixing the latter,
c. producing a wet granulation,
d. drying the granules,
e. sieving dried granules,
f. mixing the granules with magnesium stearate and silicium dioxide,
g. compressing the granules into tablets, and
h. film-coating the tablets with a water-soluble agent.

16. A method for preparing a pharmaceutical combination formulation according to any of the preceding claims, comprising the steps of
a. adding lactose, polyvinylpyrrolidone, colloidal silicon dioxide, and magnesium stearate to vildagliptin and glimepiride, and stirring the resulting mixture,
b. compressing the granules into tablets, and
c. film-coating the tablets with a water-soluble agent.

17. The pharmaceutical combination formulation according to any of the preceding claims for preventing or treating diabetes mellitus in mammalians and particularly in humans.

## Patentansprüche

1. Pharmazeutische Kombinations-Formulierung, umfassend Vildagliptin oder ein pharmazeutisch akzeptables Salz von Vildagliptin, Glimepirid oder ein pharmazeutisch akzeptables Salz von Glimepirid und mindestens einer Überzugsschicht.

2. Pharmazeutische Kombinations-Formulierung nach einem der vorangehenden Ansprüche, wobei die Überzugsschicht ein versiegelter Überzug ist, der mindestens eines oder eine Mischung von Polyvinylalkohol und Hydroxypropylmethylcellulose umfasst.

3. Pharmazeutische Kombinations-Formulierung nach einem der vorangehenden Ansprüche, ferner umfassend mindestens eines oder eine Mischung von Xanthan und Guargummi im Überzug.

4. Pharmazeutische Kombinations-Formulierung nach einem der vorangehenden Ansprüche, wobei das Gewicht der Überzugsbeschichtung in Bezug auf das Gesamtgewicht der Formulierung 0,01 bis 20 Gewichts-% ist, bevorzugt 0,1 bis 10 Gewichts-%, und besonders bevorzugt 1 bis 5 Gewichts-%.

5. Pharmazeutische Kombinations-Formulierung nach einem der vorangehenden Ansprüche, ferner umfassend mindestens einen oder mehr als einen Hilfsstoff.

6. Pharmazeutische Kombinations-Formulierung nach einem der vorangehenden Ansprüche, wobei der Hilfsstoff mindestens eines oder eine Mischung aus Verdünnungsmitteln, Bindemitteln, Sprengmitteln, oberflächenaktiven Stoffen, Gleitmitteln und Schmiermitteln ist.

7. Pharmazeutische Kombinations-Formulierung nach einem der vorangehenden Ansprüche, wobei die Verdünnungsmittel mindestens eines oder eine Mischung aus Laktose, mikrokristalliner Cellulose, Stärke, Mannitol, Calciumphosphat-Anhydrat, Calciumphosphat-Dihydrat, Calciumphosphat-Trihydrat, dibasischem Calciumphosphat, Calciumcarbonat, Calciumsulfat, Carboxymethyl-Cellulose-Calcium, pulverförmiger Cellulose, Celluloseacetat, vorgelatinierter Stärke, Laktose-Monohydrat, Maisstärke umfassen, wobei das bevorzugte Verdünnungsmittel mikrokristalline Cellulose oder Laktose ist.

8. Pharmazeutische Kombinations-Formulierung nach einem der vorangehenden Ansprüche, wobei die Bindemittel mindestens eines oder eine Mischung aus Polymethacrylat, Glycerylbehenat, Polyvinylpyrrolidon (Povidon), Hydroxypropylmethylcellulose (HPMC), Hydroxypropylcellulose (HPC), Carboxymethylcellulose (CMC), Methylcellulose (MC), Hydroxyethylcellulose, Natriumcarboxymethylcellulose (Na-CMC), Carboxymethylcellulose-Calcium, Ethylcellulose und anderen Cellulose-Derivaten, Polyethylenoxid, Gelatine, Stärke, Xanthan, Guargummi, Alginat, Carrageen, Pectin, Carbomer, Celluloseacetatphthalat, Hydroxypropyl-Stärke, Hydroxyethylmethyl-Cellulose, Polaxomer, Polyethylenglycol (PEG) umfassen, wobei das bevorzugte Bindemittel Polyvinylpyrrolidon ist.

9. Pharmazeutische Kombinations-Formulierung nach einem der vorangehenden Ansprüche, wobei die Sprengmittel mindestens eines oder eine Mischung aus Croscarmellose-Natrium, Natrium-Stärke-Glykolat, quervernetztem Polyvinylpyrrolidon, Stärke, Alginsäure umfasst, wobei das bevorzugte Sprengmittel Croscarmellose-Natrium ist.

10. Pharmazeutische Kombinations-Formulierung nach einem der vorangehenden Ansprüche, wobei die oberflächen-aktiven Stoffe mindestens eines oder eine Mischung aus Natriumlaurylsulfat, Polyoxyethylenglycolestern, Sulfaten umfassend oberflächenaktive Stoffe und Poloxamer-Derivate (Sulfat und Poloxamer-Derivate, die oberflächenaktive Stoffe umfassen) umfassen.

11. Pharmazeutische Kombinations-Formulierung nach einem der vorangehenden Ansprüche, wobei die Gleitmittel mindestens eines oder eine Mischung aus kolloidalem Siliciumdioxid, Talk, Aluminiumsilikat, pulverförmiger Cellulose, tribasischem Calciumphosphat, hydrophober kolloidaler Silica, Magnesiumoxid, Magnesium-Trisilikat, Magnesiumsilikat umfassen, wobei das bevorzugte Gleitmittel kolloidales Siliciumdioxid ist.

12. Pharmazeutische Kombinations-Formulierung nach einem der vorangehenden Ansprüche, wobei die Schmiermittel mindestens eines oder eine Mischung aus Calciumstearat, Magnesiumstearat, Mineralöl, Natriumstearylfumarat, Talk, Polyethylenglycol, Glycerylmonostearat, Glycerylpalmitostearat, Talk, Zinkstearat, Stearinsäure umfassen, wobei das bevorzugte Schmiermittel Magnesiumstearat ist.

13. Pharmazeutische Kombinations-Formulierung nach einem der vorangehenden Ansprüche, ferner umfassend cellulosisches Polymer im Kern und Xanthan oder Guargummi in der Überzugsschicht.

14. Pharmazeutische Kombinations-Formulierung nach einem der vorangehenden Ansprüche, wobei das cellulosische Polymer bevorzugt Hydroxypropylmethylcellulose ist.

15. Verfahren zur Herstellung einer pharmazeutischen Kombinations-Formulierung nach einem der vorangehenden Ansprüche, umfassend die Schritte
A. Zusetzen von Verdünnungsmittel zu Vildagliptin und Glimepirid, Mischen der resultierenden Mischung,
b. Zugeben von Polyvinylpyrrolidon und quervernetztem Polyvinylpyrrolidon zur resultierenden Mischung und Mischen der letzteren,
c. Herstellen einer Nassgranulierung,
d. Trocknen des Granulats,
e. Absieben des getrockneten Granulats,
f. Mischen des Granulats mit Magnesiumstearat und Siliciumdioxid,
g. Pressen des Granulats in Tabletten, und
h. Beschichten der Tabletten mit einem wasser-löslichen Stoff.

16. Verfahren zur Herstellung einer pharmazeutischen Kombinations-Formulierung nach einem der vorangehenden Ansprüche, umfassend die Schritte
a. Zugeben von Laktose, Polyvinylpyrrolidon, kolloidalem Siliciumdioxid und Magnesiumstearat zu Vildagliptin und Glimepirid, und Rühren der resultierenden Mischung,
b. Pressen des Granulats in Tabletten, und
c. Beschichten der Tabletten mit einem wasser-löslichen Stoff.

17. Pharmazeutische Kombinations-Formulierung nach einem der vorangehenden Ansprühe zum Verhindern oder Behandeln von Diabetes mellitus in Säugetieren und vorzugsweise in Menschen.

## Revendications

1. Formulation de combinaison pharmaceutique comprenant de la vildagliptine ou un sel pharmaceutiquement acceptable de vildagliptine, du glimépiride ou un sel pharmaceutiquement acceptable de glimépiride et au moins un agent d'enrobage.

2. Formulation de combinaison pharmaceutique selon l'une quelconque des revendications précédentes, dans laquelle ladite couche d'enrobage est un enrobage étanche comprenant au moins un ou un mélange de d'alcool polyvynilique et d'hydroxypropyle methyle cellulose.

3. Formulation de combinaison pharmaceutique selon l'une quelconque des revendications précédentes comprenant en outre au moins un ou un mélange de gomme de xanthane et de gomme de guar dans l'enrobage.

4. Formulation de combinaison pharmaceutique selon l'une quelconque des revendications précédentes dans laquelle le poids de ladite couche d'enrobage par rapport au poids total de la formulation est de 0,01 à 20% en poids, préférentiellement 0,1 à 10 % en poids, et plus préférentiellement 1 à 5% en poids.

5. Formulation de combinaison pharmaceutique selon l'une quelconque des revendications précédentes comprenant en outre au moins un ou plus d'un excipient.

6. Formulation de combinaison pharmaceutique selon l'une quelconque des revendications précédentes dans laquelle ledit excipient est au moins un ou un mélange de diluants, des liants, des agents de délitement, des agents de surface, des agents de glissement et des lubrifiants.

7. Formulation de combinaison pharmaceutique selon l'une quelconque des revendications précédentes dans laquelle lesdits diluants incluent au moins un ou un mélange de lactose, de cellulose microcristalline, d'amidon, de mannitol, de phosphate de calcium anhydre, du dihydrate de phosphate de calcium, du trihydrate de phosphate de calcium, de phosphate de calcium dibasique, de carbonate de calcium, de sulfate de calcium, de calcium carbométhyle cellulose, de cellulose pulvérisée, d'acétate de cellulose, d'amidon prégélatinisé, de monohydrate de lactose, de fécule de maïs, le diluant préféré étant la cellulose microcristalline ou le lactose.

8. Formulation de combinaison pharmaceutique selon l'une quelconque des revendications précédentes dans laquelle lesdits liants incluent au moins un ou un mélange de polyméthylacrylate, de béhénate de glycéryle, de polyvinylpyrrolidone (povidone), d'hydroxypropyle méthyle cellulose (HPMC), d'hydroxypropyle cellulose (HPC), de carboxyméthyle cellulose (CMC), de méthyle cellulose (MC), d'hydroxyéthyle cellulose, de carboxyméthyle cellulose de sodium (Na CMC), de calcium de carboxyméthyle cellulose, de l'éthyle cellulose ou d'autres dérivés de la cellulose, l'oxyde de polyéthylène, de la gélatine, de l'amidon, de la gomme de xanthane, de la gomme guar, de l'alginate, de la mousse d'Irlande, de la pectine, du carbomère, du phtalate d'acétate de cellulose, de l'amidon hydroxypropyle, de l'hydroxyéthyle cellulose, du polyaxomère, du polyéthylène glycol (PEG), les liants étant préférentiellement de la polyvinylpirrolidone.

9. Formulation de combinaison pharmaceutique selon l'une quelconque des revendications précédentes dans laquelle lesdits agents de délitement incluent au moins un ou un mélange de sodium de croscamellose, de glycolate d'amidon sodique, de polyvinylpyrrolidone réticulé, d'amidon, d'acide alginique, l'agent de délitement préféré étant le sodium de croscamellose.

10. Formulation de combinaison pharmaceutique selon l'une quelconque des revendications précédentes dans laquelle lesdits agents de surface incluent au moins un ou un mélange de sulfate de lauryle sodique, d'esters de polyoxyéthylène glycol, d'agents de surface comprenant des sulfates et des dérivés de poloxamères (dérivés de sulfate et de polaxomères comprenant des agents de surface).

11. Formulation de combinaison pharmaceutique selon l'une quelconque des revendications précédentes dans laquelle lesdits agents de glissement incluent au moins un ou un mélange de dioxyde de silice colloïdale, du talc, du silicate d'aluminium, de la cellulose pulvérisée, du phosphate de calcium tribasique, de la silice colloïdale hydrophobe, de l'oxyde de magnésium, du trisilicate de magnésium, du silicate de magnésium, l'agent de glissement préféré étant le dioxyde de silice colloïdale.

12. Formulation de combinaison pharmaceutique selon l'une quelconque des revendications précédentes dans laquelle lesdits lubrifiants incluent au moins un ou un mélange de stéarate de calcium, de stéarate de magnésium, d'huile minérale, de fumarate de stérayle sodique, de polyéthylène glycol, de monostéarate de glycéryle, de palmitostéarate de glycéryle, de talc, de stéarate de zinc, d'acide stéarique, le lubrifiant préféré étant le stéarate de magnésium.

13. Formulation de combinaison pharmaceutique selon l'une quelconque des revendications précédentes comprenant en outre un polymère cellulosique dans le coeur et de la gomme de xanthane ou de la gomme guar dans la couche d'enrobage.

14. Formulation de combinaison pharmaceutique selon l'une quelconque des revendications précédentes dans laquelle ledit polymère cellulosique est préférentiellement de l'hydroxypropyle méthyle cellulose.

15. Méthode pour préparer une formulation de combinaison pharmaceutique selon l'une quelconque des revendications précédentes, comprenant les étapes:
a. d'ajout de diluants dans la vildaglipine et la glimepiride, et le mélange du mélange résultant,
b. d'ajout de polyvinylpyrrolidone et de polyvinylpirrolidone réticulée au mélange résultant et le mélange de ce dernier,
c. de production d'une granulation humide,
d. de séchage des granules,
e. de tamisage des granules séchés,
f. de mélange des granules avec du stéarate de magnésium et du dioxyde de silicium,
g. de compression des granules en comprimés, et
h. d'enrobage par un film des comprimés avec un agent soluble dans l'eau.

16. Méthode pour préparer une formulation de combinaison pharmaceutique selon l'une quelconque des revendications précédentes, comprenant les étapes:
a. d'addition de lactose, de polyvinylpyrrolidone, de dioxyde de silicone colloïdal et de stéarate de magnésium à la vildaglipine et la glimepiride, et l'agitation du mélange résultant,
b. de compression des granules en comprimés, et
c. d'enrobage par un film des comprimés avec un agent soluble dans l'eau.

17. Formulation de combinaison pharmaceutique selon l'une quelconque des revendications précédentes pour la prévention ou le traitement des diabètes sucrés chez les mammifères, et en particulier chez l'homme.
